# EUROPÄISCHE PATENTSCHRIFT

(11) **EP 0 934 084 B1**
(45) Veröffentlichungstag und Bekanntmachung des Hinweises auf die Patenterteilung: **20.06.2001**
(21) Anmeldenummer: 97912188.6
(22) Anmeldetag: 15.10.1997
(51) Int. Cl.: A61L 2/18

(54) **VERWENDUNG EINER WÄSSRIGEN CHLORITLÖSUNG ZUR DESINFEKTION IN DER ERNÄHRUNGSINDUSTRIE**
USE OF AN AQUEOUS CHLORITE SOLUTION FOR DISINFECTION PURPOSES IN THE FOOD INDUSTRY
UTILISATION DE SOLUTION DE CHLORITE AQUEUSE A DES FINS DE DESINFECTION DANS L'INDUSTRIE ALIMENTAIRE

(30) Priorität: 24.10.1996 DE 19644251
(43) Veröffentlichungstag der Anmeldung: 11.08.1999
(73) Patentinhaber: HENKEL-ECOLAB GmbH & CO. OHG, 40589 Düsseldorf (DE)
(72) Erfinder: KLUSCHANZOFF, Harald, D-40822 Mettmann (DE); GROSSE BÖWING, Walter, PL-31-503 Krakau (PL)
(74) Vertreter: Wacker, Manfred, Dr.
(86) Internationale Anmeldenummer: EP9705687
(87) Internationale Veröffentlichungsnummer: WO9817326

(56) Entgegenhaltungen:
- WO-A-91/03265
- DE-A- 3 403 631
- DE-A- 3 928 747
- DE-A- 3 932 341
- US-A- 2 071 091
- US-A- 4 084 747
- H.F. BOHNER ET AL: "Effective Control of Microbial Populations in Polysulfone Ultrafiltration Membrane Systems" JOURNAL OF DAIRY SCIENCE., Bd. 73, Nr. 9, September 1990, CHAPAIGN, ILLINOIS US, Seiten 2309-2317, XP000200022

## Beschreibung

Die Erfindung betrifft die Desinfektion von Geräten und Behältern wie Tanks, Kegs, Flaschen und dergleichen sowie Rohrleitungen in der Ernährungsindustrie, unter Verwendung von wäßriger Chloritlösung als Desinfektions mittel.

Im Stand der Technik werden Tanks in der Ernährungsindustrie mit einem der folgenden Verfahren gereinigt. Nach einem eventuellen Entspannen bzw. Ausblasen mit Luft wird zunächst vorgespült. Danach kann sich ein alkalischer Reinigungsschritt anschließen, in welchem der Tank mit 2 %-iger Natronlauge mit oder ohne Zusatz von Additiven gereinigt wird. Dieser Reinigungsschritt, der auch entfallen kann, wird durch Zwischenspülen beendet. Danach werden die Tanks mit einer sauren Reinigungsflüssigkeit während einer Zeitdauer von 30 bis 60 min gereinigt. Das Reinigungsmittel enthält anorganische oder organische Säuren. Nach einem weiteren Zwischenspülen, das etwa 5 min dauert, wird während einer Zeitdauer von etwa 15 bis 20 min desinfiziert. Es ist aber auch möglich, daß die Desinfektion während des sauren Reinigungsschrittes vorgenommen wird, so daß das zuletzt genannte Zwischenspülen und das Desinfizieren als separate Reinigungsschritte entfallen. In einem letzten Schritt wird schließlich mit Frischwasser über eine Zeitdauer von etwa 10 min nachgespült.

Zur Desinfektion werden in den heute üblichen Reinigungs- und Desinfektionsverfahren der Ernährungsindustrie Produkte auf der Basis von Monobromessigsäure, Peressigsäure, Aktiv-Chlor, Aktivsauerstoff, quartäre Ammoniumverbindungen (QAV) und andere Verbindungen eingesetzt. Keines der genannten Biozide ist in jeglicher Hinsicht zufriedenstellend. Monobromessigsäure hat eine hohe Toxizität. Peressigsäure ist nicht problemlos handhabbar und weist Wirkungslücken gegenüber manchen Mikroorganismen auf. Aktiv-Chlor neigt zu AOX-Bildung und wird daher immer seltener eingesetzt. Quartäre Ammoniumverbindungen sind aufgrund ihres hydrophoben Charakters nachteilig.

Aus dem Stand der Technik ist ferner die Verwendung von wäßrigen Lösungen von Chlordioxid zur Desinfektion bekannt (EP 0 489 776 B1). Durch Mischen einer Lösung von Natriumchlorit und einer sauren Komponente in einem separaten Generator oder Reaktor wird eine Lösung von Chlordioxid hergestellt und unmittelbar danach zur Desinfektion eingesetzt. Derartige Generatoren sind bekannt und im Handel erhältlich. Nachteilig ist das Erfordernis eines gesonderten Gerätes zum Herstellen der desinfizierenden Lösung.

Aus der DE 34 03 631 C2 ist die Verwendung einer stabilisierten, alkalisch eingestellten wäßrigen Chloritlösung zur Aufbereitung von Wasser zu Trinkwasserzwecken sowie von Schwimmbadwasser und auch zum Entkeimen von Betriebswasser bekannt. Wird eine solche Chloritlösung einem zu behandelnden wäßrigen System zugegeben, das Chlor oder Mineralsäuren enthält, so wird hauptsächlich Chlordioxid gebildet. In der zu behandelnden Lösung vorhandene Enzyme des pflanzlichen und tierischen Stoffwechsels werden spontan durch Chlordioxid zerstört. Im Gegensatz zu den Angaben in der genannten Patentschrift wirkt Chlordioxid tatsächlich nicht mit hoher Selektivität, sondern es hat im Vergleich mit anderen Bioziden ein ausgesprochen breites Wirkungsspektrum.

Ein wesentlicher Nachteil des bekannten Verfahrens liegt in der Notwendigkeit, die zuzugebende Desinfektionslösung vor der Verwendung aus mindestens zwei Komponenten, der alkalischen Lösung und der Chloritlösung und gegebenenfalls weiteren Komponenten, herzustellen.

Im Journal of Dairy Science, Vol 73, No. 9 (September 1990), Seiten 2309 bis 2317, H.F. Bohner und R.L. Bradley, "Effektive Control of Microbial Populations in Polysulfone Ultrafiltration Membrane Systems", wird die Reinigung und Desinfektion eines Ultrafiltration-Gerätes, das in der Nahrungsmittelindustrie Verwendung findet, beschrieben. Hierbei wird im Anschluß an einen sauren Reinigungsschritt ein Chlordioxid-"Sanitizer" durch das Gerät geleitet, welcher aus zwei Komponenten besteht: einer wäßrigen Natriumchlorit-Lösung sowie einem "Aktivator", enthaltend Michlsäure und ein Tensid.

Die WO-A-91/03265 betrifft ein Verfahren zur Desinfektion harter Oberflächen, insbesondere von Geräten der Nahrungsmittelindustrie, mit Chlordioxid und entspricht der vorstehend zitierten EP-B-0 489 776.

In US-A-4,084,747 wird ein Verfahren zur Desinfektion von beispielsweise harten Oberflächen beschrieben, wobei man die Oberflächen mit einem Gemisch von Verbindungen, enthaltend Chlordioxid, in Kontakt bringt, welches in situ durch Reaktion von Natriumchlorit mit einer sauren Komponente, vorzugsweise Milchsäure, resultiert.

DE-A-39 32 341 betrifft ein Verfahren zur Sterilisation von Getränkeflaschen mit einem Wasser-Chlordioxid-Gemisch. Dieses Gemisch wird zuvor in einem Reaktor aus verdünnter Salzsäure und einer Natriumchlorit-Lösung hergestellt.

In US-A-2,071,091 werden fungizide und bakterizide Formulierungen, bestehend aus chloriger Säure oder deren löslicher Salze, insbesondere Natriumchlorit, beschrieben, die in saurem Medium ihre Wirkung entfalten.

Der Erfindung liegt die Aufgabe zugrunde, die eingangs genannte Desinfektion so durchzuführen, daß weder ein gesonderter Reaktor noch eine aus mehreren Komponenten bestehende Desinfektionslösung erforderlich ist.

Die Erfinder haben nun festgestellt, daß Chlorite mit Vorteil zur Desinfektion bei der Reinigung von Behältern und Geräten der Ernährungsindustrie und dergleichen eingesetzt werden können. Gibt man eine neutrale, keine zusätzlichen Komponenten enthaltende Chloritlösung während eines dem sauren Reinigungsschritt nachfolgenden Spülschrittes der Spülflüssigkeit zu, so wird auf einfache und wirtschaftliche Weise eine hervorragende Desinfektion erreicht. Bei der Zugabe der Chloritlösung zur Spülflüssigkeit wird ein ausreichend niedriger pH-Wert durch die an den Geräten und Behältern bzw. Rohrleitungen noch anhaftende Säure aus dem vorhergehenden sauren Reinigungsschritt erreicht. Ein zusätzliches Ansäurern ist daher nicht notwendig. Erfindungsgemäß ist es sogar in einigen Fällen möglich, auf den letzten Nachspülschritt zu verzichten, wenn die Chlordioxid-Konzentration nach Beendigung der Desinfektion unter 0,2 ppm liegt.

Gegenstand der Erfindung ist daher die Verwendung einer wäßrigen Chloritlösung in der Ernährungsindustrie zur Desinfektion während der Reinigung von Geräten und Behältern wie Tanks, Kegs, Flaschen und dergleichen sowie Rohrleitungen, die mit einer sauren Reinigungsflüssigkeit gereinigt werden, dadurch gekennzeichnet, daß man die Chloritlösung ohne vorherige Ansäuerung während eines diesem Reinigungsschritt nachfolgenden Spülschrittes der Spülflüssigkeit zugibt, wobei lediglich die nach dem sauren Reinigungsschritt noch im Gerät anhaftende Säure zur Freisetzung von Chlordioxid dient.

Ein besonderer Vorteil des erfindungsgemäßen Verfahrens liegt in der Möglichkeit, eine neutrale Chloritlösung einzusetzen, ohne daß weitere Komponenten erforderlich wären. Der das Freisetzen von Chlordioxid bewirkende niedrige pH-Wert wird durch die zur Reinigung ohnehin verwendete übliche saure Lösung erreicht.

Vorzugsweise enthält die Spülflüssigkeit 1 bis 1000 ppm, insbesondere 50 bis 400 ppm Chlorit.

Bevorzugt wird eine Lösung von Natriumchlorit eingesetzt, aber auch Kaliumsalze und andere Salze können verwendet werden.

Zu den Einwirkungszeiten wird insbesondere vorgeschlagen, daß man die chlorithaltige Spülflüssigkeit über eine Zeitdauer von mindestens 20 s und vorzugsweise von 20 s bis 20 min auf die Behälter einwirken läßt.

Dabei ist es von Vorteil, wenn die chlorithaltige Spülflüssigkeit eine Temperatur bis 40 °C und vorzugsweise eine Temperatur von 5 bis 30 °C aufweist.

Der pH-Wert der chlorithaltigen Spülflüssigkeit muß während des Desinfektionsschrittes unter 7 liegen und hat vorzugsweise einen Wert unterhalb von 5. Eine zusätzliche Säurezugabe ist jedoch nicht notwendig, da der pH-Wert der sauren Reinigungsflüssigkeit ausreichend niedrig ist und die Spülflüssigkeit durch die an den Behältern bzw. Geräten oder Rohrleitungen anhaftenden Reste aus dem vorhergegangenen sauren Reinigungsschritt ausreichend sauer ist.

Ein weiterer Gegenstand der Erfindung ist die Verwendung einen wäßrigen Chloritlösung in der Ernährungsindustrie zur Desinfektion von Geräten und Behältern wie Gär- und Lagertanks in einer kohlendioxidhaltigen Atmosphäre, wobei das CO₂ zu einer Ansäuerung des Wassers und zur ClO₂-Freisetzung führt. Gär- und Lagertanks von Brauereien werden heutzutage nämlich oft in einer Kohlendioxid-Atmosphäre gereinigt. Dabei wird die Reinigungslösung in vielen Fällen derart verschmutzt, so daß die notwendige Desinfektion in einem getrennten, nachfolgenden Schritt erfolgen sollte. Erfindungsgemäß wird hierzu eine wäßrige Lösung von Natriumchlorit durch die Tanks gepumpt. Das in der Tankatmosphäre enthaltene Kohlendioxid sorgt für eine Ansäuerung des Wassers, so daß Chlordioxid freigesetzt wird. Auch hier wird in vorteilhafter Weise eine nur aus einer Komponente bestehende Lösung von Chlorit in Wasser eingesetzt. Auch der weitere erfindungsgemäße Vorteil wird hier erreicht, daß ein Reaktor zur Freisetzung von Chlordioxid nicht erforderlich ist.

## Patentansprüche

1. Verwendung einer wäßrigen Chloritlösung in der Ernährungsindustrie zur Desinfektion während der Reinigung von Geräten und Behältern wie Tanks, Kegs, Flaschen und dergleichen sowie Rohrleitungen, die mit einer sauren Reinigungsflüssigkeit gereinigt werden,
**dadurch gekennzeichnet,**
daß man die Chloritlösung ohne vorherige Ansäuerung während eines diesem Reinigungsschritt nachfolgenden Spülschrittes der Spülflüssigkeit zugibt, wobei lediglich die nach dem sauren Reinigungsschritt noch im Gerät anhaftende Säure zur Freisetzung von Chlordioxid dient.

2. Verwendung nach dem vorhergehenden Anspruch,
**dadurch gekennzeichnet,**
daß die Spülflüssigkeit 1 bis 1000 ppm, insbesondere 50 bis 400 ppm Chlorit enthält.

3. Verwendung nach einem der vorhergehenden Ansprüche,
**dadurch gekennzeichnet,**
daß man eine Lösung von Natriumchlorit einsetzt.

4. Verwendung nach einem der vorhergehenden Ansprüche,
**dadurch gekennzeichnet,**
daß man die chlorithaltige Spülflüssigkeit über eine Zeitdauer von mindestens 20 s und vorzugsweise von 20 s bis 20 min auf die Behälter einwirken läßt.

5. Verwendung nach einem der vorhergehenden Ansprüche,
**dadurch gekennzeichnet,**
daß die chlorithaltige Spülflüssigkeit eine Temperatur bis 40 °C und vorzugsweise eine Temperatur von 5 bis 30 °C aufweist.

6. Verwendung nach einem der vorhergehenden Ansprüche,
**dadurch gekennzeichnet,**
daß der pH-Wert der chlorithaltigen Spülflüssigkeit während des Desinfektionsschrittes unter 7 und vorzugsweise unter 5 liegt.

7. Verwendung einer wäßrigen Chloritlösung in der Ernährungsindustrie zur Desinfektion von Geräten und Behältern wie Gär- und Lagertanks in einer kohlendioxidhaltigen Atmosphäre, wobei das CO₂ zu einer Ansäuerung des Wassers und zur ClO₂-Freisetzung führt.

## Claims

1. The use of an aqueous chlorite solution in the food industry for disinfection during the cleaning of equipment and containers, such as tanks, kegs, bottles and the like, and pipelines using an acidic cleaning liquid, characterized in that the chlorite solution is added without preacidification to a rinsing liquid which is used after the acidic cleaning step during a following rinsing step whereby the acidic residues in the equipment, coming from the acidic cleaning step, support the generation of chlorine dioxide.

2. The use claimed in the preceding claim, characterized in that the cleaning or rinsing liquid contains 1 to 1000 ppm and, more particularly, 50 to 400 ppm of chlorite.

3. The use claimed in any of the preceding claims, characterized in that a solution of sodium chlorite is used.

4. The use claimed in any of the preceding claims, characterized in that the chlorite-containing cleaning or rinsing liquid is allowed to act on the containers for at least 20 s and preferably for 20 s to 20 mins.

5. The use claimed in any of the preceding claims, characterized in that the chlorite-containing cleaning or rinsing liquid has a temperature of up to 40°C and, preferably, in the range from 5 to 30°C.

6. The use claimed in any of the preceding claims, characterized in that the pH value of the chlorite-containing cleaning or rinsing liquid during the disinfecting step is below 7 and preferably below 5.

7. The use of an aqueous chlorite solution in the food industry for disinfecting equipment and containers, such as fermentation and storage tanks, in an atmosphere containing carbon dioxide, the CO₂ leading to acidification of the water and to the release of ClO₂.

## Revendications

1. Utilisation d'une solution aqueuse de chlorite dans l'industrie alimentaire, pour la désinfection au cours du nettoyage des appareils et des récipients, tels que cuves, fûts, bouteilles et récipients similaires, ainsi que des canalisations, qui sont nettoyés avec un liquide de nettoyage acide,
caractérisée en ce que
l'on ajoute la solution de chlorite au liquide de rinçage sans acidification préalable au cours d'une étape de rinçage faisant suite à cette étape de nettoyage, l'acide encore adhérent dans l'appareil après l'étape de nettoyage acide étant suffisant pour libérer le dioxyde de chlore.

2. Utilisation selon la revendication qui précède,
caractérisée en ce que
le liquide de rinçage renferme 1 à 1000 ppm, en particulier 50 à 400 ppm de chlorite.

3. Utilisation selon une des revendications qui précèdent,
caractérisée en ce que
l'on utilise une solution de chlorite de sodium.

4. Utilisation selon une des revendications qui précèdent,
caractérisée en ce que
l'on laisse agir sur les récipients le liquide de rinçage contenant du chlorite pendant une période d'au moins 20 secondes et de préférence comprise entre 20 secondes et 20 minutes.

5. Utilisation selon une des revendications qui précédent,
caractérisée en ce que
le liquide de rinçage contenant du chlorite présente une température allant jusqu'à 40 °C et de préférence une température comprise entre 5 et 30 °C.

6. Utilisation selon une des revendications qui précédent,
caractérisée en ce que
le pH du liquide de rinçage contenant du chlorite est inférieur à 7 et de préférence inférieur à 5 pendant l'étape de désinfection.

7. Utilisation d'une solution aqueuse de chlorite dans l'industrie alimentaire pour la désinfection des appareils et des récipients, tels que cuves de fermentation et de stockage dans une atmosphère renfermant du dioxyde de carbone, le CO₂ entraînant une acidification de l'eau et la libération de ClO₂.
